# EUROPEAN PATENT APPLICATION

(11) **EP 3 511 411 A1**
(43) Date of publication of application: **17.07.2019**
(21) Application number: 17848822.7
(22) Date of filing: 07.09.2017
(51) Int. Cl.: C12N 5/0786, A61K 35/15, A61P 43/00

(54) **METHOD FOR PREPARING MONONUCLEAR CELLS**

(30) Priority: 09.09.2016 JP 2016176379
(71) Applicant: Yonemitsu, Yoshikazu, Fukuoka 8100023 (JP); GAIA BioMedicine Inc., Chuo-ku Fukuoka-City Fukuoka 810-0023 (JP)
(72) Inventor: YONEMITSU, Yoshikazu, Fukuoka-shi Fukuoka 810-0023 (JP); HARADA, Yui, Fukuoka-shi Fukuoka 819-0395 (JP)
(74) Representative: Vossius & Partner Patentanwälte Rechtsanwälte mbB
(86) International application number: PCT/JP2017/032186
(87) International publication number: WO 2018/047884

(57) **Abstract**

The object is to remove thrombocytes from apheresis blood. Mononuclear cells are prepared so as not to contain thrombocytes. The present invention provides a method for preparing mononuclear cells, which comprises (1) the step of mixing a non-cytotoxic and nonionic density-adjusting agent with apheresis blood to adjust density of plasma to be 1.066 to 1.078 g/ml, and (2) the step of centrifuging stratified layers including the following layers a and b to separate mononuclear cells:
a. a layer of a density gradient centrifugation medium for separation of mononuclear cells,
b. a layer of the density-adjusted apheresis blood layered under the layer a.

## Description

### Technical Field

The present invention relates to a method for preparing mononuclear cells from apheresis blood.

### Background Art

Dendritic cells can stimulate or suppress the immune system including both the acquired immunity and natural immunity as antigen-presenting cells. Therefore, there are attempted dendritic cell transplant therapies, in which dendritic cells treated outside the body of a patient beforehand so that they present a specific antigen relevant to a cancer, infectious disease, autoimmune disease, allergic disease, or the like are transplanted to the patient to control the immune system of the patient. The dendritic cells used for the therapies cannot be directly separated from the inside of the body. Therefore, dendritic cells are obtained by separating monocytes or mononuclear cells containing monocytes from blood collected from a patient, and culturing them to make them differentiate into dendritic cells (Patent document 1, etc.).

Some methods for separating monocytes or mononuclear cells including monocytes from blood have been investigated. For example, Patent document 2 proposes a mononuclear cell separation tube comprising a centrifugation tube containing a high density liquid layer of which specific gravity is adjusted to 1.064 to 1.079 g/ml, and an insoluble hydrogel layer layered so as to enclose the high density liquid layer, and comprising hydrogel made water-insolube with divalent cations as the main ingredient. Patent document 3 also proposes a mononuclear cell separation tube comprising a centrifugation tube containing a medium for separation of mononuclear cells containing a hydrogel made water-insolube with divalent cations as the main ingredient, of which specific gravity is adjusted to 1.064 to 1.079 g/ml.

Further, Patent document 4 proposes a method for separating and collecting monocytes comprising flowing a mononuclear cell suspension into a filter apparatus filled with a filter that captures monocytes, but passes lymphocytes, and then flowing a collected liquid into the filter apparatus to collect monocytes, wherein the mononuclear cell suspension contains 1000 to 10000 cells as total nucleated cell number per 1 mm² of the surface area of the filter. Patent document 5 proposes a mononuclear cell separation tube for centrifuging a sample liquid containing two or more kinds of components including mononuclear cells using a centrifugation medium to separate two or more kinds of the components on the basis of difference of specific gravity and collecting mononuclear cells that constitute a low specific gravity component, which comprises an upper chamber having an introduction opening that is provided on the upper side, through which the sample liquid is introduced, and a lower side opening provided on the lower side, in which the sample liquid is contained before the centrifugation, and mononuclear cells that constitute a low specific gravity component are contained after the centrifugation, a lower chamber having an upper side opening on the upper side and a closed lower side, in which the centrifugation medium is stored before the centrifugation, and high specific gravity components as unnecessary components are contained after the centrifugation, a stopper cock that switches communicable state and non-communicable state between the lower side opening of the upper chamber and the upper side opening of the lower chamber, and a lid for sealing the introduction opening of the upper chamber.

### Prior Art References

### Patent documents

Patent document 1: Japanese Patent Unexamined Publication (KOKAI) No. 2016-94458
Patent document 2: Japanese Patent Unexamined Publication (KOKAI) No. 5-34338
Patent document 3: Japanese Patent Unexamined Publication (KOKAI) No. 5-34339
Patent document 4: Japanese Patent Unexamined Publication (KOKAI) No. 2004-129550
Patent document 5: Japanese Patent Unexamined Publication (KOKAI) No. 2012-34725

### Non-patent documents

Non-patent document 1: M. Kobayashi et al., Dendritic Cell-Based Immunotherapy Targeting Synthesized Peptides for Advanced Biliary Tract Cancer, J. Gastrointest. Surg., 2013 Sep; 17(9):1609-17
Non-patent document 2: H. Takahashi et al., Impact of dendritic cell vaccines pulsed with Wilms' tumour-1 peptide antigen on the survival of patients with advanced non-small cell lung cancers, European Journal of Cancer (2013), 49, 852-859
Non-patent document 3: M. Kobayashi et al., Prognostic factors related to add-on dendritic cell vaccines on patients with inoperable pancreatic cancer receiving chemotherapy: a multicenter analysis, Cancer Immunol. Immunother., 2014 Aug; 63(8):797-806
Non-patent document 4: S. Mayanagi et al., Phase I pilot study of Wilms tumor gene 1 peptide-pulsed dendritic cell vaccination combined with gemcitabine in pancreatic cancer, Cancer Sci., 2015 Apr; 106(4):397-406

### Disclosure of the Invention

### Problem to be Solved by the Invention

There are methods for producing dendritic cells for transplant therapies, which comprise culturing monocytes obtained by separation from blood obtained with an apheresis apparatus (apheresis blood) (Non-patent documents 1 to 4). Since the apheresis blood contains blood cell components other than monocytes, mononuclear cells (lymphocytes and monocytes) are collected from the apheresis blood by density gradient centrifugation. Then, the collected mononuclear cells are put on a plate so that the monocytes are adhered by the plate, and the monocytes are thereby separated. In this process, since thrombocytes mixed in the mononuclear cells inhibit the adhesion of the monocytes to the plate, the mononuclear cells are usually put on the plate after removing the thrombocytes by repeatedly performing centrifugal washing. In the washing process, monocytes are lost at the same time with the removal of the thrombocytes, and therefore the methods have a problem of the reduction of the number of dendritic cells as the final object.

### Means for Solving the Problem

The inventors of the present invention investigated methods for stably preparing cells for therapeutic use such as dendritic cells with high quality, and conducted various researches in order to solve the aforementioned problem by preparing mononuclear cells not containing thrombocytes. As a result, they found that when cells of apheresis blood are separated by density gradient centrifugation, by appropriately adjusting density of the apheresis blood, the aforementioned problem can be solved, and accomplished the present invention.

The present invention provides the followings.
[1] A method for preparing mononuclear cells, the method comprising:
   (1) the step of mixing a non-cytotoxic and nonionic density-adjusting agent with apheresis blood to adjust density of plasma to be 1.066 to 1.078 g/ml, and
   (2) the step of centrifuging stratified layers including the following layers a and b to separate mononuclear cells:
      a. a layer of a density gradient centrifugation medium for separation of mononuclear cells,
      b. a layer of the density-adjusted apheresis blood layered under the layer a.
[2] The method according to claim 1, wherein the stratified layers further include the following layer c:
   c. a layer of an isotonic solution layered on the top of the layer a.
[3] The method according to claim 1 or 2, wherein the density-adjusting agent is an iodinated contrast medium.
[4] The method according to claim 3, wherein the iodinated contrast medium is iodixanol.
[5] The preparation method according to any one of claims 1 to 3, wherein the layer b is a layer of apheresis blood of which density of plasma is adjusted to 1.069 to 1.078 g/ml.
[6] The method according to any one of claims 1 to 3, wherein the layer b is a layer of apheresis blood of which density of plasma is adjusted to 1.073 to 1.078 g/ml.
[7] The method according to any one of claims 1 to 3, wherein the density of plasma of the layer b is adjusted by mixing 20 to 22 parts by volume of a 60 w/v % iodixanol aqueous solution with 100 parts by volume of the apheresis blood.
[8] The method according to any one of claims 1 to 7, wherein the density gradient centrifugation medium for separation of mononuclear cells contains polysucrose 400 and sodium diatrizoate, and the density of plasma is 1.072 to 1.085 g/ml.
[9] A method for preparing useful cells, the method comprising the steps defined in any one of claims 1 to 8, and which further comprises (3) the step of obtaining useful cells from the prepared mononuclear cells.
[10] The method according to claim 9, wherein the useful cells are any cells selected from the group consisting of dendritic cells, NK cells, NKT cells, activated T cells, and CAR-T cells.

### Effect of the Invention

According to the present invention, mononuclear cells from which thrombocytes are sufficiently removed can be obtained from apheresis blood at a high recovery ratio.

### Brief Description of the Drawings

[Fig. 1] Results of flow cytometry analysis of mononuclear cells.
[Fig. 2] Microphotographs of (1) the sample obtained with 2.0 ml of OptiPrep, and (2) positive control. Magnifications are 4 times for the upper row, and 20 times for the lower row.
[Fig. 3] Removal efficiency for thrombocytes.
[Fig. 4] Results of flow cytometry analysis of mononuclear cells.
[Fig. 5] Results of flow cytometry analysis of mononuclear cells.
[Fig. 6] Results of flow cytometry analysis of mononuclear cells.
[Fig. 7] Change of cell number over 14 days.

### Modes for Carrying out the Invention

The ranges of numerical values indicated as "X to Y" include the numerical values of X and Y as the minimum and maximum values, unless especially indicated. The expression "A and/or B" means at least one of A and B, unless especially indicated.

The unit of density is g/ml, unless especially indicated, and the values thereof are those observed at 20°C. The term density used for a fluid has the same meaning as that of specific gravity. The symbol "%" and the term "part" may be based on volume, mass, or both. Preparation method is production method in other words.

The present invention relates to a method for preparing mononuclear cells, which comprises the following steps (1) and (2):
(1) the step of adjusting density of plasma of apheresis blood to be 1.078 g/ml or lower, and
(2) the step of centrifuging stratified layers comprising the following layers a and b to separate mononuclear cells:
   a. a layer of a density gradient centrifugation medium for separation of mononuclear cells,
   b. a layer of the density-adjusted apheresis blood layered under the layer a.

### [Step (1): Density adjustment step]

The step (1) is a step of adjusting the density of apheresis blood. Apheresis generally means separation of plasma components and cell components from blood by extracorporeal circulation. Apheresis blood refers to blood collected by extracorporeal circulation, and it is usually continuously subjected to an anticoagulation treatment during the extracorporeal circulation, and contains an anticoagulation ingredient. Examples of the anticoagulation ingredient include EDTA (ethylenediaminetetraacetic acid), heparin, sodium citrate, sodium fluoride, and ACD (acid citrate dextrose solution). In a preferred embodiment, the apheresis blood contains an ACD-A solution. The ACD-A solution contains 2.20 w/v % of sodium citrate hydrate, 0.80 w/v % of citric acid hydrate, and 2.20 w/v % of glucose as active ingredients. At the time of apheresis, the ACD-A solution is usually used at a ratio of 15 ml per 100 ml of blood volume. In a particularly preferred embodiment, the apheresis blood contains the ACD-A solution at this ratio.

According to the present invention, the density of the plasma (liquid component in the collected blood) of the apheresis blood is adjusted with a non-cytotoxic and nonionic density-adjusting agent. Since the agent is nonionic and can penetrate through cell membranes, by mixing it with the apheresis blood, densities of not only plasma, but also blood cell components can be adjusted. Several kinds of agents that are non-cytotoxic, i.e., not toxic to blood cells, and nonionic, and can adjust density of blood or plasma are well known by those skilled in the art, and are also marketed.

Preferred examples of the non-cytotoxic and nonionic density-adjusting agent are iodinated contrast mediums. The iodinated contrast mediums include iohexanol, iodixanol, ioversol, iopamidol, iotrolan, metrizamide, iodecimol, ioglucol, ioglucamide, ioglunide, iogulamide, iomeprol, iopentol, iopromide, iosarcol, iosimide, iotusal, ioxilan, iofrotal, iodecol, and derivatives of these.

In a preferred embodiment, the density of the plasma of the apheresis blood can be adjusted by using iodixanol. Iodixanol (IUPAC name is 5-{N-[3-(N-{3,5-bis[(2,3-dihydroxypropyl)carbamoyl]-2,4,6-triiodophenyl}acetamido)-2-hydroxypropyl]acetamido}-1-N,3-N-bis(2,3-dihydroxypropyl)-2,4,6-triiodobenzene-1,3-dicarboxamide) can be mixed with the apheresis blood as an aqueous solution of an appropriate concentration, for example, a 40 to 80 w/v % aqueous solution. Iodixanol is marketed as a sterilized aqueous solution. For example, OptiPrep (registered trademark, Axis-Shield), which is a sterilized 60 w/v % iodixanol aqueous solution, can also be preferably used for the present invention. The solution of iodixanol may further contain sodium calcium edetate hydrate, trometamol, sodium chloride, or calcium chloride for the purpose of adjustment of osmotic pressure, or the like, and may contain a pH modifier. In this specification, the present invention may be explained for an embodiment in which iodixanol is used as the non-cytotoxic and nonionic density-adjusting agent as an example, but those skilled in the art can appropriately apply the explanation to the cases using other non-cytotoxic and nonionic density-adjusting agents, and easily understand such cases. The layer of the apheresis blood of which density of plasma is adjusted is referred to as layer b in the present invention.

The density is adjusted so that the density of the plasma of the apheresis blood becomes appropriate. In general, specific gravity of the whole peripheral blood is usually 1.057 (1.055 to 1.06) in the case of males, or 1.053 (1.050 to 1.056) in the case of females, and specific gravity of the plasma of peripheral blood is about 1.027 (1.025 to 1.029), although they may change depending on protein concentration, hematocrit value, hemoglobin content, and so forth. The density of the plasma in the apheresis blood can be easily obtained on the basis of this usual value of the density of plasma, 1.027 g/ml, and the value of the density of the ACD-A solution, 1.008 g/ml, by calculation. When the ACD-A solution is used in the standard volume, i.e., when the ACD-A solution is used in a volume of 15 ml for 100 ml of the blood volume, the density of the plasma of a standard apheresis blood is calculated to be about 1.025 g/ml.

In the layer b, the nonionic density-adjusting agent is mixed in the apheresis blood, and therefore the density of plasma is adjusted so as to be higher than that of a standard apheresis blood, and adjusted to such a density that the layer b can be stratified under the layer a, which will be described later, i.e., a density higher than that of the layer a. Such a density is, specifically, 1.066 g/ml or higher. As for some of the methods of the prior art, it is disclosed that, for the separation of mononuclear cells from peripheral blood, the density of plasma should be adjusted to be 1.063 to 1.066 g/ml. However, according to the investigations of the inventors of the present invention, even if this density range is applied to apheresis blood, mononuclear cells cannot be separated. Specifically, if an iodixanol solution is added so that the density of plasma should be within the aforementioned range on the basis of calculation, the layers a and b cannot be stratified as the upper layer and lower layer, respectively. It is considered that this is because the specific gravity of apheresis blood differs from that of peripheral blood. In addition, mixing of a larger volume of iodixanol solution may not necessarily provide a satisfactory result. A too large volume results in a density of plasma larger than the density of erythrocytes, and in such a case, the plasma constitutes the lowest layer after centrifugation. Moreover, since iodixanol permeates into hemocytes, the density of mononuclear cells also rises, thus the layer containing mononuclear cells is not formed on the layer a even after centrifugation, and accordingly, mononuclear cells cannot be collected.

Therefore, in the layer b, the density of plasma should be adjusted to be 1.078 g/ml or lower. In a preferred embodiment, the density of plasma in the layer b is adjusted to be 1.069 to 1.078 g/ml, more preferably 1.073 to 1.078 g/ml.

In a particularly preferred embodiment, in the layer b, the density is adjusted by mixing 20 to 22 parts by volume of a 60 w/v % iodixanol aqueous solution with 100 parts by volume of the apheresis blood.

According to the present invention, a density gradient centrifugation medium for separation of mononuclear cells is used as the layer a. Various density gradient centrifugation mediums for separation of mononuclear cells from blood are marketed, and well known to those skilled in the art. Any of these can be used for the present invention. In a preferred embodiment, the density gradient centrifugation medium for separation of mononuclear cells contains polysucrose 400 and sodium diatrizoate, and has a density of 1.072 to 1.085 g/ml. As such a density gradient centrifugation medium for separation of mononuclear cells, Ficoll PM400, Ficoll-Paque PLUS, Percoll PLUS, Percoll, Ficoll-Paque PREMIUM, and so forth are marketed (all from GE Healthcare).

The volume of the layer a with respect to the layer b is not particularly limited, so long as the centrifugation operation described later can be performed, and a layer containing objective mononuclear cells can be appropriately collected after the centrifugation. When a marketed material is used as the layer a, it may be used with conditions for obtaining peripheral blood mononuclear cells (PBMCs) described in the attached instructions. For example, 0.50 to 2.0 parts by volume, preferably 0.66 to 1.5 parts by volume, more preferably 0.66 to 1.3 parts by volume, of the layer a can be used for 1 part by volume of the layer b.

In the present invention, a layer of an isotonic solution (layer c) may be stratified on the layer a. By stratifying the layer c, it becomes easy to collect the objective layer after centrifugation. As the isotonic solution, there can be used physiological saline, Ringer's solution (lactated Ringer's solution, acetic acid Ringer's solution, bicarbonate Ringer's solution, etc.), and 5% glucose aqueous solution, as well as those having a buffering effect, such as phosphate buffered saline (PBS), Tris-buffered saline (TBS), HEPES-buffered saline, basal mediums for animal cell culture (DMEM, EMEM, RPMI-1640, α-MEM, F-12, F-10, M-199, etc.), and so forth.

The volume of the layer c is not particularly limited, so long as the centrifugation operation can be performed, and the objective layer can be appropriately collected after the centrifugation. For example, 0.01 to 10 parts by volume, preferably 0.05 to 2 parts by volume, more preferably 0.1 to 1 part by volume, of the layer c can be used for 1 part by volume of the layer b.

The layers a, b, and the layer c, as required, are subjected to the centrifugation step to be explained below in a state that the layer c (if used), the layer a, and the layer b are stratified in this order from the top. The order of providing these layers is not limited, so long as they can be stratified in such a state as described above. Typically, the layer a is first filled in a centrifugation tube, then the layer b is filled under the layer a by using a pipet, or the like, and if necessary, the layer c is layered as the uppermost layer, i.e., on the layer a.

### [Step (2): Centrifugation step]

The step (2) is a step of centrifuging the stratified layers including at least the layer a and the layer b to separate mononuclear cells. In the step (2), the stratified layers including at least the layer a and the layer b are subjected to a centrifugation operation at 15 to 30°C and a centrifugal acceleration of 400 to 2000 g for about 10 to 40 minutes. By this centrifugation operation, the layer b is divided into a layer containing mononuclear cells (also referred to as PBMC layer) and another layer, the former is formed on the layer a, and the latter is formed under the layer a.

After the centrifugation, the layer containing mononuclear cells can be collected into another tube by using a pipet 12 or the like, and mononuclear cells can be collected from the layer. The collected mononuclear cells may be washed by using an appropriate isotonic solution, if needed, and may be made into a cell suspension diluted to an appropriate cell density with an isotonic solution. Examples of the isotonic solution that can be used in this operation are the same as those mentioned above for the layer c. Those skilled in the art can obtain the recovery amount and recovery ratio of the mononuclear cells by well-known methods.

The operations of the steps (1) and (2) of the method of the present invention can be performed manually, or the method can applied to a processing apparatus used in the manufacturing process of products for regeneration medicine, etc. Examples of such a processing apparatus include the blood component separation system, COM.TEC., produced by Fresenius Kabi Japan.

Although the present invention relates to a method for preparing mononuclear cells from apheresis blood, it can be expected to be applied to peripheral blood, cord blood, and a fluid containing blood cells collected from bone marrow or lymph gland, so long as the density of plasma can be adjusted as in the case of apheresis blood.

### [Advantages of the present invention, and characteristics of mononuclear cells and monocytes to be obtained]

According to the present invention, mononuclear cells can be collected from apheresis blood at a recovery ratio equivalent to or higher than those obtainable by conventional methods. Moreover, the obtained mononuclear cells are characterized by little contamination with thrombocytes. Such little contamination with thrombocytes can be evaluated by, for example, measuring nucleated cell ratio, granulocyte contamination ratio etc. with a flow cytometer. The nucleated cell ratio is represented as (number of nucleated cells in the objective cell population)/(total cell number of the objective cell population) x 100, and a higher value of this ratio indicates less contamination with thrombocytes or erythrocytes. The granulocyte contamination ratio is represented as (cell number of granulocytes in the objective cell population)/(total cell number in the objective cell population) x 100, and a lower value of this ratio indicates a higher mononuclear cell ratio.

By using the method of the present invention, the nucleated cell ratio can be made to be 40% or higher, and the ratio can be made to be 80% or higher in a preferred embodiment, or 85% or higher in a more preferred embodiment. By using the method of the present invention, the granulocyte contamination ratio can be made to be 0.7% or lower, and the ratio can be made to be 0.4% or lower in a preferred embodiment, or 0.33% or lower in a more preferred embodiment.

By using the method of the present invention, sufficient amount of monocytes can be collected with little contamination with thrombocytes. That is, the ratio of monocytes contained in the obtained mononuclear cells (monocyte ratio) is not inferior to those obtainable by the conventional methods. The ratio of monocytes in mononuclear cells can be evaluated by, for example, measuring monocyte ratio with a flow cytometer. The monocyte ratio is represented as (cell number of monocytes in the objective cell population)/(total cell number of the objective cell population) x 100. In a preferred embodiment, a monocyte ratio of 15 to 20% can be obtained.

From the mononuclear cells obtained according to the present invention, monocytes can be collected by conventional techniques. Specifically, the mononuclear cells are inoculated on a plate at an appropriate density, and incubated at 37°C for several tens of minutes to make monocytes adhere to the plate. Then, by washing the plate surface with an appropriate solution, cells that do not adhere can be removed. Since the mononuclear cells obtained by the method of the present invention are contaminated with few thrombocytes, the bottom surface of the plate is hardly covered with thrombocytes, and a sufficient amount of mononuclear cells can be made to adhere to the plate, and obtained.

In general, thrombocytes contained in apheresis blood are concentrated 2 to 5 times compared with the peripheral blood (number of thrombocytes is usually 1.40 to 3.40 x10⁸ ml). Therefore, if the removal efficiency for the thrombocytes contaminating mononuclear cells can be increased by even in an order of about 0.1%, absolute number of the thrombocytes contaminating mononuclear cells can be markedly reduced, and such reduction has great technical significance. According to the investigations of the inventors of the present invention, the thrombocyte removal efficiencies obtainable by the methods of the prior art are around 99.30%. In such cases, when mononuclear cells obtained after the centrifugation are inoculated on a plate, adhesion of thrombocytes to the plate is conspicuous, and it can be microscopically confirmed that the ratio of the monocytes covering the bottom surface of the plate is low. For obtaining monocytes by the plate method, the removal efficiency for thrombocytes in the mononuclear cells collected after the centrifugation is preferably 99.50% or higher. This is because if thrombocytes are removed to such an extent, adhesion of thrombocytes to the plate is not conspicuous, and the substantially whole surface of the plate can be covered with monocytes. The removal efficiency for thrombocytes is more preferably 99.65% or higher, still more preferably 99.80% or higher. Such a high removal efficiency for thrombocytes can easily be achieved by a preferred embodiment of the present invention. The thrombocyte removal efficiency is represented as [(number of thrombocytes in apheresis blood) - (number of thrombocytes contaminating obtained mononuclear cells)]/(number of thrombocytes in apheresis blood) x 100.

Prolongation of the time of contact with thrombocytes may also provide bad influences. Monocytes obtained by the conventional methods, which are contaminated with many thrombocytes, may provide increase of side scatter light (SSC) in flow cytometry. This is considered to indicate that phagocytosis occurs due to the long-term contact with thrombocytes in the separation step. According to the method of the present invention, which provides high thrombocyte removal efficiency, such a bad influence can be reduced.

In the mononuclear cells or monocytes prepared according to the present invention, not only contamination with thrombocytes but also contamination with neutrophiles is suppressed compared with those observed with the conventional methods.

### [Use of mononuclear cells and monocytes]

The mononuclear cells and monocytes prepared according to the present invention can be used for various uses. For example, various useful cells can be obtained from the mononuclear cells or monocytes prepared according to the present invention. That is, the present invention also provides a method for preparing useful cells. The useful cells are not particularly limited, so long as they are obtained from the prepared mononuclear cells. Examples of the useful cells include cells useful for treatment of a disease or condition of human or non-human animal or elucidation of the mechanism thereof, cells useful for search of drug candidate compounds for human or non-human animal, and cells useful for evaluation of toxicity, and evaluation of environmental influence.

The method for preparing useful cells may further comprise, in addition to the aforementioned steps (1) and (2), the following step (3):
(3) the step of obtaining useful cells from the prepared mononuclear cells.

Examples of the cells useful for treatment of a disease or condition of human or nonhuman animal include dendritic cell, NK cell (natural killer cell), NKT cell (natural killer T cell), activated T cell (including Th1 cell, Th2 cell, T_{FH} cell, Th17 cell, Treg cell, etc., which are members of a functional subgroup of the effecter T cells produced as a result of activation of naive T cells at the time of encountering an antigen), and CAR-T cell (chimeric antigen receptor T cell). Particularly preferred examples include dendritic cell and NK cell.

In the method for preparing dendritic cells, the aforementioned step (3) may be the following step (3-1) or (3-2).
(3-1) the step of separating monocytes from the prepared mononuclear cells, and culturing the separated monocytes to obtain dendritic cells,
(3-2) the step of culturing the prepared mononuclear cells to obtain dendritic cells.

The method for preparing dendritic cells may further comprises the step of removing T cells from the mononuclear cells, and removal of T cells in this step may be performed by removing cells expressing CD3. The culture performed in the step (3-1) or (3-2) may be performed in a medium containing GM-CSF and SCF, this culture may be performed for at least 3 weeks, preferably 4 weeks, and the culture may then be performed after changing the medium to a medium containing GM-CSF and IL-4. As for the detailed culture conditions, and so forth, Patent document 1 mentioned above can be referred to.

In the method for preparing NK cells, the aforementioned step (3) may be the following step (3-3):
(3-3) the step of culturing the prepared mononuclear cells to obtain NK cells.

In the method for preparing NK cells, the NK cells may be obtained by culturing hemopoietic precursor cells contained in the prepared mononuclear cells, and inducing differentiation thereof, or the NK cells may be obtained by culturing NK cells contained in the prepared mononuclear cells to proliferate them. In the former case, the medium used in the culture of the step (3-3) preferably contains IL-15, SCF, IL-7, and F1t3L, and the medium more preferably further contains TPO. The amplified hemopoietic precursor cells may be further cultured with the culture conditions including use of IL-2. As for the detailed culture conditions, and so forth, Japanese Patent No. 5511039 can be referred to.

In the latter case, the medium used for the culture in the step (3-3) preferably contains IL-2. The method may further comprise the step of removing T cells, and in such a case, the removal of T cells may be performed by removing cells expressing CD3. The method may further comprise the step of removing hemopoietic precursor cells, and in such a case, the removal of hemopoietic precursor cells may be performed by removing CD34-positive cells. For the detailed culture conditions etc., Japanese Patent No. 5572863 can be referred to.

For the preparation of NKT cells, for example, the following reference can be referred to: Motohashi S, Kobayashi S, Ito T, Magara KK, Mikuni O, Kamada N, Iizasa T, Nakayama T, Fujisawa T, Taniguchi M., Preserved IFN-alpha production of circulating Valpha24 NKT cells in primary lung cancer patients, Int. J. Cancer, 2002 Nov 10;102(2):159-65 (http://onlinelibrary.wiley.com/doi/10.1002/ijc.10678/abstract).

For the preparation of activated T cells, for example, the following reference can be referred to: Schellhorn M, Haustein M, Frank M, Linnebacher M, Hinz B, Celecoxib increases lung cancer cell lysis by lymphokine-activated killer cells via upregulation of ICAM-1, Oncotarget., 2015 Nov 17;6(36):39342-56, doi: 10.18632/oncotarget.5745 (https://www.ncbi.nlm.nih.gov/pmc/articles/PMC4770776/)

For the preparation of CAR-T cells, for example, the following reference can be referred to: Tumaini B, Lee DW, Lin T, Castiello L, Stroncek DF, Mackall C, Wayne A, Sabatino M, Simplified process for the production of anti-CD 19-CAR-engineered T cells, Cytotherapy, 2013 Nov; 15(11):1406-15, doi: 10.1016/j.jcyt.2013.06.003.Epub 2013 Aug 28 (https://www.ncbi.nlm.nih.gov/pmc/articles/PMC4141724/)

The diseases and conditions to which the prepared cells useful for treatment of a disease or condition of human or nonhuman animal can be applied include cancers, infectious diseases, autoimmune diseases, allergies, and so forth. For the production of dendritic cells for transplant therapies, methods of culturing monocytes obtained by separation from apheresis blood are well known (Non-patent documents 1 to 4 mentioned above), and the method of the present invention can also be preferably applied to these methods.

The prepared cells useful for treatment of a disease or condition of human or nonhuman animal can constitute a product for regeneration medicine, or the like. Therefore, the present invention also provides a method for producing a pharmaceutical composition (including product for regeneration medicine or the like) containing the cells useful for treatment of a disease or condition of human or nonhuman animal. The pharmaceutical composition may contain a pharmaceutically acceptable additive, besides the useful cells as the active ingredient.

### Examples

### [Example 1: Separation of mononuclear cells from apheresis blood of healthy person (manual operation)]

Separation of blood components was performed for blood of healthy volunteer collected by apheresis using ACD-A solution (TERUMO) on the apheresis system COM.TEC. produced by Fresenius Kabi Japan (MNC: Mononuclear cell collection was used as the program) (henceforth referred to as apheresis blood) by using OptiPrep (60 w/v % iodixanol solution, density 1.320 ± 0.001 g/ml, Axis-Shield) and Ficoll (Ficoll-Paque PLUS, density 1.077 ± 0.001 g/ml, GE Healthcare).

1. Fill 15 ml of Ficoll in a 50 ml conical tube produced by Nunc (henceforth referred to as 50 ml tube) (prepared for 5 tubes).
2. Similarly fill 10 ml of the apheresis blood in another 50 ml tube (prepared for 5 tubes).
3. Add 1.6 ml, 1.8 ml, 2.0 ml, and 2.2 ml of OptiPrep to 4 tubes obtained in 2 mentioned above, respectively, and mix it with the blood to obtain one tube for each OptiPrep addition volume.
4. Fill each of the mixtures of the apheresis blood and OptiPrep prepared in 3 mentioned above under each Ficoll layer prepared in 1 mentioned above using a 25 ml pipet.
5. Stratify 5 ml of PBS as the uppermost layer of the liquid in each of the 50 ml tubes prepared in 4 mentioned above (on the Ficoll layer).
6. Prepare a tube by stratifying 2-fold diluted apheresis blood (prepared by adding 10 ml of PBS to the blood prepared in 2 mentioned above) as an upper layer on the Ficoll layer of the tube prepared in 1 mentioned above as a positive control.
7. Centrifuge the 50 ml tubes prepared in 5 and 6 mentioned above (500 g, room temperature, 20 minutes, no brake).
8. Collect and centrifuge each PBMC layer (500 g, 4°C, 15 minutes, with brake).
9. Discard each supernatant, and suspend the residue in 5 ml of PBS (the positive control is additionally subjected to thrombocyte removal step, i.e., subject it to centrifugation twice with 50 ml of PBS (300 g, 4°C, 5 minutes, with brake).
10. Dilute the suspension 20 times with the Turk's solution, and count cell number by using a blood cell counting plate.

The results of the counting of the cell numbers are shown in the following table. In the tube prepared by adding 1.6 ml of OptiPrep in 4 mentioned above, the mixture could not be filled as a lower layer of Ficoll, and mixed with it, and therefore it was excluded from the experiment.

**[Table 1]**

| Example 1: Numbers of collected cells | |
|---|---|
| Positive control | 1.08E+08 |
| Opti 1.6 ml | - |
| Opti 1.8 ml | 1.40E+08 |
| Opti 2.0 ml | 1.37E+08 |
| Opti 2.2 ml | 1.55E+08 |

In the groups in which 1.8 to 2.2 ml of OptiPrep was added to the apheresis blood, the numbers of the collected cells were equivalent to or higher than that of the positive control. This means that, by calculations, it is impossible to perform separation of mononuclear cells by adjusting the addition amount of OptiPrep in consideration of difference of specific gravities of the apheresis blood and peripheral blood in light of the description of the OptiPrep application sheet C05 (http://www.axis-shield-density-gradient-media.com/C05.pdf) that it is necessary to adjust specific gravity of liquid component of blood to be between 1.063 and 1.066 (following table).

**[Table 2]**

| Specific gravity chart | | | | | | | | |
|---|---|---|---|---|---|---|---|---|
| | Plasma: ACD-A solution | 100:100 | 50:100 | 100:50 | 100:20 | 100:15 | 100:0 | 0:100 |
| | Apheresis blood plasma | 1.017000000 | 1.014333333 | 1.020666667 | 1.023833333 | 1.024521739 | 1.027000000 | 1.008000000 |
| Apheresis blood plasma 10+ | 0pt1.25 | 1.050666667 | 1.048296296 | 1.053925926 | 1.056740741 | 1.057352657 | 1.059555556 | 1.042666667 |
| | 0pt1.4 | 1.054210526 | 1.051871345 | 1.057426901 | 1.060204678 | 1.060808543 | 1.062982456 | 1.046315789 |
| | 0pt1.538676608(C05) | 1.057404895 | 1.055093827 | 1.060582613 | 1.063327006 | 1.063923614 | 1.066071400 | 1.049605040 |
| | 0pt1.6 | 1.058793103 | 1.056494253 | 1.061954023 | | | 1.067413793 | 1.051034483 |
| | 0pt1.8 | 1.063220339 | 1.060960452 | 1.066327684 | 1.069011299 | 1.069594694 | 1.071694915 | 1.055593220 |
| | 0pt2.0 | 1.067500000 | 1.065277778 | 1.070555556 | *1.073194444* | *1.073768116* | 1.075833333 | 1.060000000 |
| | 0pt2.2 | 1.071639344 | 1.069453552 | 1.074644809 | *1.077240437* | *1.077804704* | 1.079836066 | 1.064262295 |
| | 0pt2.5 | 1.077600000 | 1.075466667 | 1.080533333 | 1.083066667 | 1.083617391 | | 1.070400000 |
| | | 1.095555556 | 1.093580247 | 0pt3.5 1.098271605 | 1.100617284 | 1.101127214 | | 1.088888889 |

| | | | | | | | | |
|---|---|---|---|---|---|---|---|---|
| Underlines indicate values specified in the application sheet C05 and calculated values. The values in the box are values with which experimental separation is possible. Strikethroughs means values with which experimental separation is impossible. Italic numbers are optimal values according to the present invention (explained later). | | | | | | | | |

According to the application sheet C05, for peripheral blood (column of 100:0 in the table), mononuclear cells can be collected by adding OptiPrep in a volume of 1.4 ml (separation is experimentally possible, specific gravity is adjusted to 1.0630) to 1.539 ml (specific gravity is adjusted to 1.0661), and therefore in order to perform similar adjustment of specific gravity for the apheresis blood (column of 100:20 or 100:15 in the table), it is necessary to add OptiPrep in a volume of 1.539 ml (specific gravity is adjusted to 1.0639) to 1.6 ml (specific gravity is adjusted to 1.0647) per 10 ml of the apheresis blood. However, if 1.6 ml of OptiPrep is added to the apheresis blood, the mixture cannot be filled as a lower layer of the Ficoll layer (the specific gravity of the whole blood is equivalent to or lower than the specific gravity of Ficoll, 1.077, and therefore centrifugation cannot be performed.

Then, the nucleated cell ratio (number of nucleated cells/total cell number x 100, higher value indicates lower contamination of thrombocytes and erythrocytes), granulocyte contamination ratio (cell number of granulocytes/total cell number x 100, lower value indicates higher mononuclear cell ratio), and monocyte ratio (monocyte number/total cell number x 100) in the collected cells were measured with a flow cytometer. The cells of each group collected in 9 mentioned above were put in each well of EZ-BindShutII 96-well plate (Asahi Glass) in an amount of 4 x 10⁵ cells in duplicate, and subjected to centrifugation (500 g, 4°C, 5 minutes), and the supernatant was discarded. Then, the cells were suspended in each of the following antibody solutions, and the reaction was allowed at 4°C for 30 minutes in a dark place.

### Antibody solution I: CD14-FITC 50 µl (Biolegend)

### Antibody solution II: Isotype control-FITC 50 µl (Biolegend)

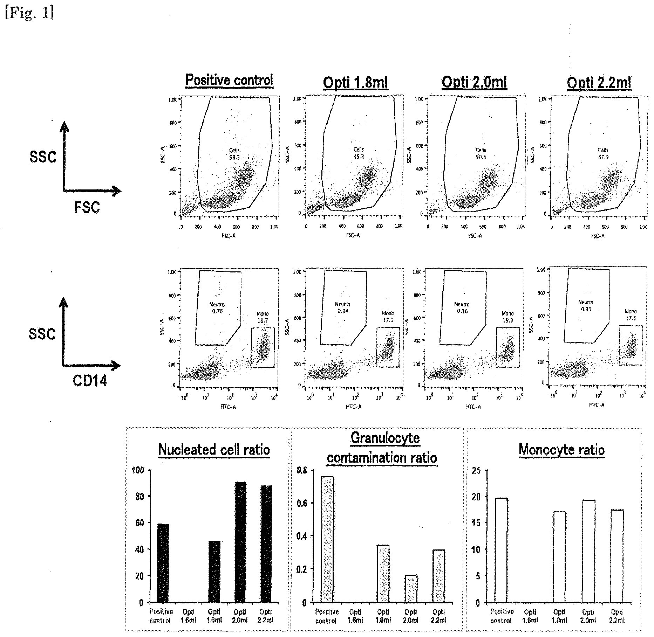

After the completion of the reaction, 200 µl of PBS was added to each well, and centrifugal washing was performed (500 g, 4°C, 5 minutes). To each well, 200 µl of PBS was added, and analysis was performed with LSRFortessa (BD Biosciences, software FACSDiva) to obtain the results shown in Fig. 1.

The nucleated cell ratio tended to become high with the OptiPrep addition volumes of 2.0 ml and 2.2 ml, the granulocyte contamination ratio was low with all the OptiPrep addition volumes of 1.8 to 2.2 ml, and the monocyte ratio was comparable to that of the positive control with all the OptiPrep addition volumes of 1.8 to 2.2 ml. These results indicate that thrombocyte-free mononuclear cells can be most efficiently separated and purified with the OptiPrep addition volumes of 2.0 ml and 2.2 ml, and the values indicated in italics in the above table are optimal.

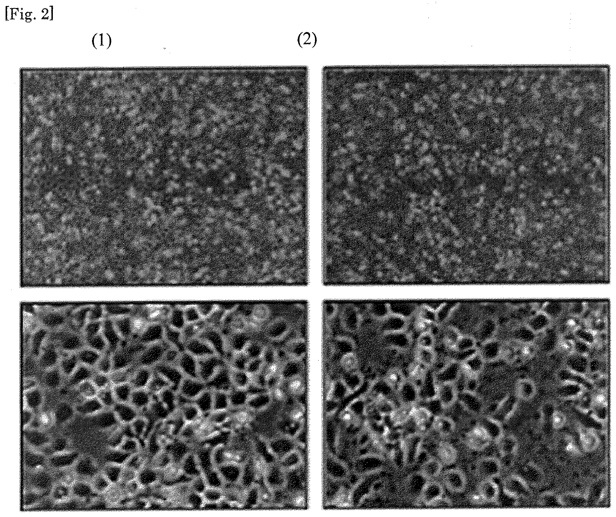

Further, the cells collected by the method that used the OptiPrep addition volume of 2.0 ml and those of the positive control were each suspended in the AIM-V medium (Thermo Fisher Scientific), inoculated on Primaria 10 cm dishes (Corning) at a density of 6 x 10⁷ cells/6 ml, and incubated at 37°C for 30 minutes, and the monocytes were thereby adhered to the dishes (general method for culturing dendritic cells used by those skilled in the art). Then, the dish surfaces were washed by using the AIM-V medium, and the cells not adhered were collected. The cells adhering to the dishes were photographed, and the observation results shown in Fig. 2 were obtained as a result. In the case of the positive control, adhesion of remaining thrombocytes was conspicuous, and reduction of the ratio of monocytes covering the bottom surface of the dish could be confirmed. On the other hand, for the OptiPrep addition group, remaining thrombocytes hardly existed, and the bottom surface was covered with monocytes.

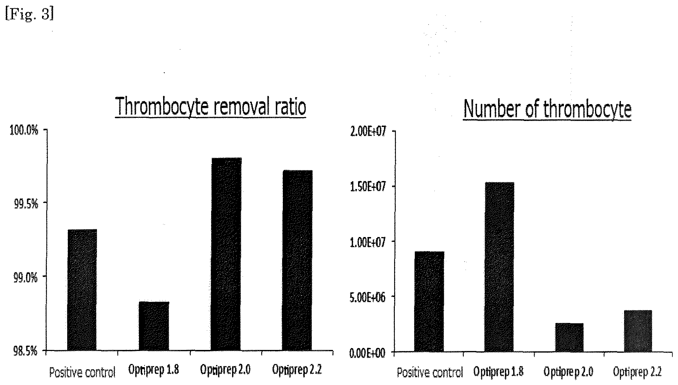

The efficiencies of the removal of thrombocytes for the experiment groups are summarized in the following table and Fig. 3. The measurements of the numbers of mononuclear cells and thrombocytes were entrusted to SRL, Co., Ltd., and the calculations were performed on the basis on those results.

**[Table 3]**

| | Apheresis blood | Positive control | Optiprep 1.8 | Optiprep 2.0 | Optiprep 2.2 |
|---|---|---|---|---|---|
| Number of mononuclear cells | 1.931E+07 | 2.16E+07 | 2.80E+07 | 2.27E+07 | 3.10E+07 |
| Number of mononuclear cells (for 10 ml of apheresis blood) | 1.93E+08 | 1.08E+08 | 1.40E+08 | 1.36E+08 | 1.55E+08 |
| Number of thrombocytes | 6.54E+08 | 9.01E+06 | 1.53E+07 | 2.56E+06 | 3.75E+06 |
| Number of thrombocytes (for 10 ml of apheresis blood) | 6.54E+09 | 4.50E+07 | 7.66E+07 | 1.28E+07 | 1.88E+07 |
| Total | 8.665E+08 | 1.386E+08 | 1.833E+08 | 1.658E+08 | 1.898E+08 |
| | | | | | |
| Ratio of mononuclear cells and thrombocytes | 2.23 | 58.3 | 45.3 | 90.6 | 87.9 |
| Removal ratio of thrombocytes | - | 99.31% | 98.83% | 99.80% | 99.71% |

| | | | | | |
|---|---|---|---|---|---|
| Liquid volume after each treatment was 5 ml. | | | | | |

The apheresis blood was highly concentrated for thrombocytes (about 2 to 5 times) compared with the peripheral blood. Therefore, it can be understood that, if the removal efficiency differs by about 1%, the number of the remaining thrombocytes markedly changes. Therefore, it was demonstrated that the thrombocyte removal efficiency is generally desirably 99.5% or higher, and in order to simultaneously achieve such a removal efficiency and minimum loss of mononuclear cells, addition of 2.0 or 2.2 ml of OptiPrep is optimal.

### [Example 2: Separation of mononuclear cells from apheresis blood of healthy person (CliniMACS Prodigy (registered trademark))]

Separation of blood components was performed for blood of healthy volunteer collected by apheresis using ACD-A solution (TERUMO) on the apheresis system COM.TEC. produced by Fresenius Kabi Japan (henceforth referred to as apheresis blood) by using OptiPrep and Ficoll.
1. Connect TS510 Tubing Set, bags containing various reagents (20 ml or more of OptiPrep, 100 of ml Ficoll, and 1,000 ml of EDTA buffer) and a bag containing 100 ml of the apheresis blood to the completely closed automatic cell preparation system, CliniMACS Prodigy (registered trademark, Miltenyi Biotec), and start the program (20 ml of OptiPrep is used for 100 ml of the apheresis blood).
2. Transfer the collected cell suspension from a collection bag to a 225 ml tube.
3. Dilute the suspension 20 times with the Turk's solution, and count cell number by using a blood cell counting plate.

The result of counting of the cell number is shown in the following table. It was demonstrated that cells can be collected in a number substantially equivalent to that obtained by the manual operation.

**[Table 4]**

| Example 2: Number of collected cells | |
|---|---|
| Prodigy | 1.14E+09 |

Then, the nucleated cell ratio (higher value indicates lower contamination of thrombocytes and erythrocytes), and granulocyte contamination ratio (lower value indicates higher mononuclear cell ratio) in the collected cells were measured with a flow cytometer. The collected cells were put in each well of EZ-BindShutII 96-well plate (Asahi Glass) in an amount of 4 x 10⁵ cells in duplicate, and subjected to centrifugation (500 g, 4°C, 5 minutes), and the supernatant was discarded. Then, the cells were suspended in each of the following antibody solutions, and the reaction was allowed at 4°C for 30 minutes.

### Antibody solution I: CD14-FITC 50 µl (Biolegend)

### Antibody solution II: Isotype control-FITC 50 µl (Biolegend)

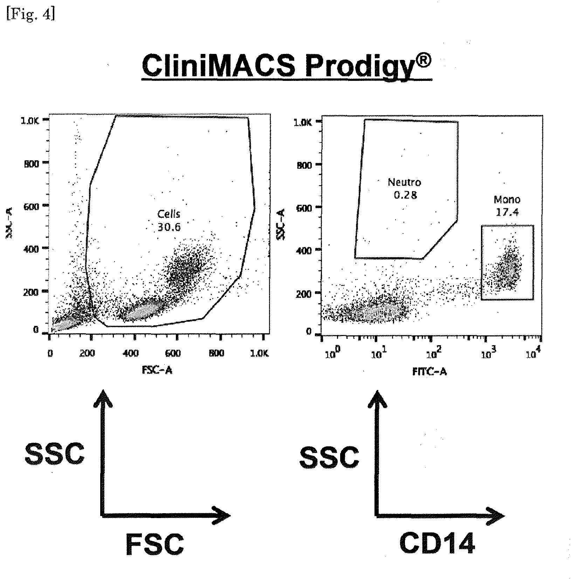

After the completion of the reaction, 200 µl of PBS was added to each well, and centrifugal washing was performed (500 g, 4°C, 5 minutes). To each well, 200 µl of PBS was added, and analysis was performed with LSRFortessa (software FACSDiva). The results are shown in Fig. 4.

### [Example 3: Separation of mononuclear cells from apheresis blood of healthy person (manual operation, after storage of blood for 24 hour)]

Separation of blood components was performed for blood of healthy volunteer collected by apheresis using ACD-A solution (TERUMO) on the apheresis system COM.TEC. produced by Fresenius Kabi Japan, and stored at 4°C for 24 hours (henceforth referred to as apheresis blood) by using OptiPrep (60 w/v % iodixanol solution, Axis-Shield) and Ficoll (GE Healthcare).
1. Fill 15 ml of Ficoll in a 50 ml conical tube produced by Nunc (henceforth referred to as 50 ml tube) (prepared for 2 tubes).
2. Similarly fill 10 ml of the apheresis blood in another 50 ml tube (prepared for 2 tubes).
3. Add 2.0 ml of OptiPrep to one tube obtained in 2 mentioned above, and mix it with the blood.
4. Fill the mixture of the apheresis blood and OptiPrep prepared in 3 mentioned above under the Ficoll layer prepared in 1 mentioned above using a 25 ml pipet.
5. Stratify 5 ml of PBS as the uppermost layer of the liquid in the 50 ml tube prepared in 4 mentioned above (on the Ficoll layer).
6. Prepare a tube by stratifying 2-fold diluted apheresis blood (prepared by adding 10 ml of PBS to the blood prepared in 2 mentioned above) as an upper layer on the Ficoll layer of the tube prepared in 1 mentioned above as a positive control.
7. Centrifuge the 50 ml tubes prepared in 5 and 6 mentioned above (500 g, room temperature, 20 minutes, no brake).
8. Collect and centrifuge each PBMC layer (500 g, 4°C, 15 minutes, with brake).
9. Discard each supernatant, and suspend the residue in 5 ml of PBS (the positive control is additionally subjected to thrombocyte removal step, i.e., subject it to centrifugation twice with 50 ml of PBS (300 g, 4°C, 5 minutes, with brake).
10. Dilute the suspension 20 times with the Turk's solution, and count cell number by using a blood cell counting plate.

The results of counting of the cell number are shown in the following table. It was demonstrated that cells can be collected in a number substantially equivalent to that obtained by the manual operation.

**[Table 5]**

| Example 3 : Number of collected cells | |
|---|---|
| Positive control | 1.60E+08 |
| Opti 2.0 ml | 1.31E+08 |

Then, the nucleated cell ratio (higher value indicates lower contamination of thrombocytes and erythrocytes), and granulocyte contamination ratio (lower value indicates higher mononuclear cell ratio) in the collected cells were measured with a flow cytometer. The cells of each group collected in 9 mentioned above were put in each well of EZ-BindShutII 96-well plate in an amount of 4 x 10⁵ cells in duplicate, and subjected to centrifugation (500 g, 4°C, 5 minutes), and the supernatant was discarded. Then, the cells were suspended in each of the following antibody solutions, and the reaction was allowed at 4°C for 30 minutes.

### Antibody solution I: CD14-FITC 50 µl (Biolegend)

### Antibody solution II: Isotype control-FITC 50 µl (Biolegend)

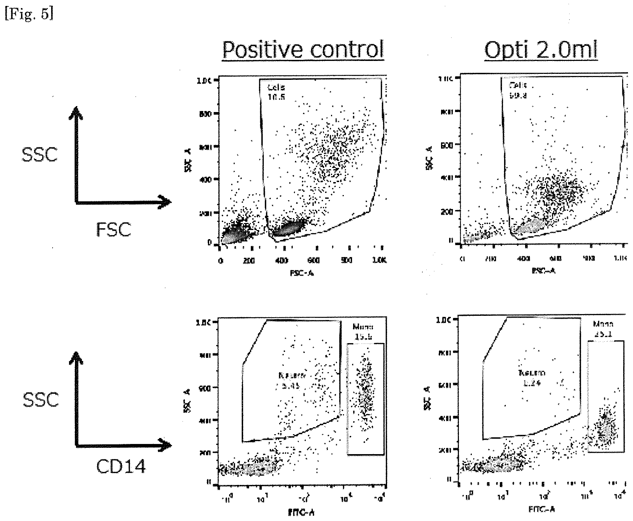

After the completion of the reaction, 200 µ1 of PBS was added to each well, and centrifugal washing was performed (500 g, 4°C, 5 minutes). To each well, 200 µl of PBS was added, and analysis was performed with LSRFortessa (software FACSDiva). The results are shown in Fig. 5.

For the positive control, marked elevation of monocyte SSC was observed, and it is considered that phagocytosis occurred due to the long-term contact with thrombocytes in the separation process. In addition, in the positive control, the contamination by neutrophiles exceeded 5%, and it was judged that the conditions are inappropriate for culture of dendritic cells.

### [Example 4: Separation of mononuclear cells from apheresis blood of healthy person (CliniMACS Prodigy (registered trademark))]

Separation of blood components was performed for blood of healthy volunteer collected by apheresis using ACD-A solution (TERUMO) on the apheresis system COM.TEC. produced by Fresenius Kabi Japan (henceforth referred to as apheresis blood) by using OptiPrep and Ficoll.
1. Connect TS510 Tubing Set, bags containing various reagents (20 ml or more of OptiPrep, 100 of ml Ficoll, and 1,000 ml of EDTA buffer) and a bag containing 100 ml of the apheresis blood to CliniMACS Prodigy (registered trademark), and start the program (20 ml of OptiPrep was used for 100 ml of the apheresis blood).
2. Transfer the collected cell suspension from a collection bag to a 225 ml tube.
3. Dilute the suspension 20 times with the Turk's solution, and count cell number by using a blood cell counting plate (PBMC).
4. Remove CD3-positive cells by using CliniMACS CD3 beads (130-050-101, Miltenyi Biotec) and LD column (130-042-901, Miltenyi Biotec).
5. Dilute the suspension 20 times with the Turk's solution, and count cell number by using a blood cell counting plate (CD3-depleted).

The results of counting of the cell number are shown in the following table.

It was demonstrated that cells can be collected in a number substantially equivalent to that obtained by the manual operation.

**[Table 6]**

| Example 4 : Number of collected cells | |
|---|---|
| PBMC | 1.23E+09 |
| CD3-depleted | 4.99E+08 |

Then, the ratio of CD30-positive cells, and the ratio of NK cells (CD3-negative, and CD56-positive) in the collected cells were measured with a flow cytometer. The cells of each group collected in 2 and 4 mentioned above were put in each well of EZ-BindShutII 96-well plate (Asahi Glass) in an amount of 4 x 10⁵ cells in duplicate, and subjected to centrifugation (500 g, 4°C, 5 minutes), and the supernatant was discarded. Then, the cells were suspended in each of the following antibody solutions, and the reaction was allowed at 4°C for 30 minutes.

### Antibody solution I: CD3-PerCP-Cy5.5, CD56-PE 50 µl (Biolegend)

### Antibody solution II: Isotype control-FITC 50 µl (Biolegend)

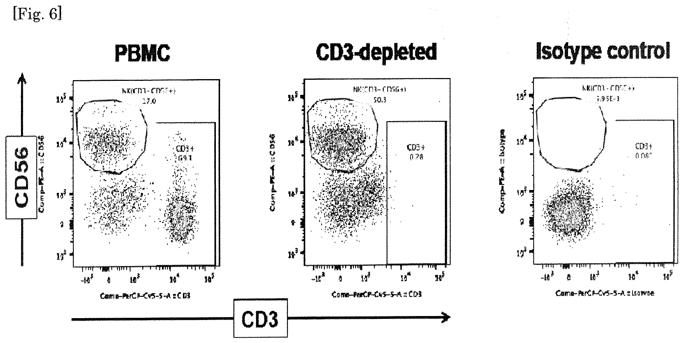

After the completion of the reaction, 200 µl of PBS was added to each well, and centrifugal washing was performed (500 g, 4°C, 5 minutes). To each well, 200 µl of PBS was added, and analysis was performed with LSRFortessa (software FACSDiva). The results are shown in Fig. 6.

Then, CD3-depleted PBMC was cultured in the following manners, and the growth ratio thereof was measured.
(1) The cells in a number of 2 x 10⁸ were transferred to a 50 mL centrifugation tube according to the number of the peripheral blood mononuclear cells after separation, and centrifugation was performed at 500 x g for 5 minutes.
(2) The supernatant formed after centrifugation was removed, and the cells were suspended in 1600 µL of an isolation buffer. CliniMACS CD3 beads (50 µL) were added and suspended in the suspension, and 80 µl of the isolation buffer and 2.5 µl of CliniMACS CD3 were added per 1 x 10⁷ cells.
(3) The reaction was allowed at 4°C for 15 minutes (the centrifugation tube was shaken every 5 minutes to suspend the cells).
(4) Alter the completion of the reaction, 40 mL of the isolation buffer was added (2 ml or more of the isolation buffer was added per 1 x 10⁷ cells), and centrifugation was performed at 300 x g for 10 minutes.
(5) The LD column was rinsed (2 ml was applied).
(6) The supernatant was removed, the cells were suspended in 1 mL of the isolation buffer, the cell suspension was applied to the rinsed LD column, and the eluted liquid was collected.
(7) Then, the column was washed twice, the number of the cells in the collected liquid was counted, and the total cell number was calculated.
(8) The liquid was centrifuged at 500 x g for 5 minutes, the supernatant was removed, then the cells were suspended in the KBM501 medium (Kohjin Bio) containing 5% human type AB serum (inactivated by a reaction at 56°C for 30 minutes, Kohjin Bio) (henceforth referred to as NK medium) at a density of 5 x 10⁵ cells/mL, and the suspension was inoculated to T-175 flask (Greiner Bio-One) in a volume of 12 mL/flask.
(9) The cells were cultured in a CO² incubator at 37°C.
(10) A part of the culture medium was taken on the 9th day of the culture, and the cell number was counted. The NK medium was added so that the cell density became 5 x 10⁵ cells/mL, and the cells were cultured in a CO² incubator at 37°C.
(11) The culture supernatant was collected on the 14th day. PBS containing 1 mM EDTA was added to the flask after the collection of the supernatant, the reaction was allowed at room temperature for 5 minutes, and the adhered cells were separated, and collected. The number of the collected cells was counted, and the total cell number was calculated. The cells were centrifuged at 500 x g for 5 minutes, the supernatant was removed, and the cells were suspended in STEM-CELL BANKER (Nippon Zenyaku Kogyo), and stored at -80°C.

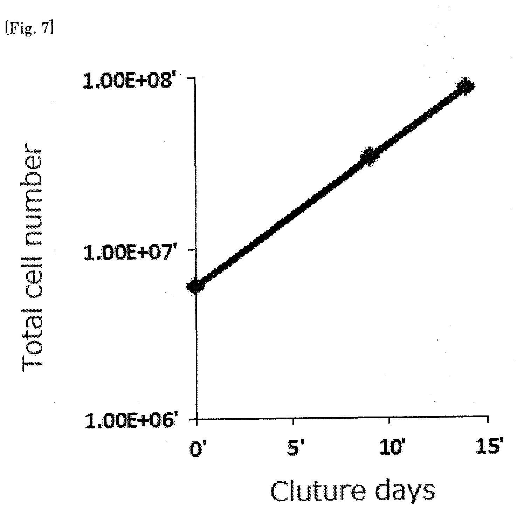

Change of the cell number during the main culture step was as shown in Fig. 7. Since these results are comparable to the data contained in the report of the inventors of the present invention (S. Saito et al., Ex Vivo Generation of Highly Purified and Activated Natural Killer Cells from Human Peripheral Blood: HUMAN GENE THERAPY METHODS, 24:241-252 (August 2013)), and therefore it was demonstrated that the method of the present invention is useful also for a method of culturing NK cells obtained by removing CD3-positive cells from prepared mononuclear cells (Method for preparing NK cells (Japanese Patent No. 5511039), and Method for amplifying NK cells (Japanese Patent No. 5572863)).

If the method of the present invention is used, the time of the treatment in CliniMACS Prodigy is markedly shortened (shortened to about 4 to 2 hours), and therefore it is extremely useful at the time of mass production in future.

## Claims

1. A method for preparing mononuclear cells, the method comprising:
(1) the step of mixing a non-cytotoxic and nonionic density-adjusting agent with apheresis blood to adjust density of plasma to be 1.066 to 1.078 g/ml, and
(2) the step of centrifuging stratified layers including the following layers a and b to separate mononuclear cells:
a. a layer of a density gradient centrifugation medium for separation of mononuclear cells,
b. a layer of the density-adjusted apheresis blood layered under the layer a.

2. The method according to claim 1, wherein the stratified layers further include the following layer c:
c. a layer of an isotonic solution layered on the top of the layer a.

3. The method according to claim 1 or 2, wherein the density-adjusting agent is an iodinated contrast medium.

4. The method according to claim 3, wherein the iodinated contrast medium is iodixanol.

5. The preparation method according to any one of claims 1 to 3, wherein the layer b is a layer of apheresis blood of which density of plasma is adjusted to 1.069 to 1.078 g/ml.

6. The method according to any one of claims 1 to 3, wherein the layer b is a layer of apheresis blood of which density of plasma is adjusted to 1.073 to 1.078 g/ml.

7. The method according to any one of claims 1 to 3, wherein the density of plasma of the layer b is adjusted by mixing 20 to 22 parts by volume of a 60 w/v % iodixanol aqueous solution with 100 parts by volume of the apheresis blood.

8. The method according to any one of claims 1 to 7, wherein the density gradient centrifugation medium for separation of mononuclear cells contains polysucrose 400 and sodium diatrizoate, and the density of plasma is 1.072 to 1.085 g/ml.

9. A method for preparing useful cells, the method comprising the steps defined in any one of claims 1 to 8, and which further comprises (3) the step of obtaining useful cells from the prepared mononuclear cells.

10. The method according to claim 9, wherein the useful cells are any cells selected from the group consisting of dendritic cells, NK cells, NKT cells, activated T cells, and CAR-T cells.
